# EUROPEAN PATENT APPLICATION

(11) **EP 4 678 206 A1**
(43) Date of publication of application: **14.01.2026**
(21) Application number: 24306156.1
(22) Date of filing: 09.07.2024
(51) Int. Cl.: A61M 5/32, A61M 5/31

(54) **SAFETY DEVICE FOR PROTECTING AGAINST NEEDLE STICK INJURIES AND INJECTION DEVICE INCLUDING SAID SAFETY DEVICE**

(71) Applicant: Becton Dickinson France, 38800 Le Pont-de-Claix (FR)
(72) Inventor: VALENTIN, Stéphane, 38470 L'ALBENC (FR); MILLS, Freddy, 38650 SINARD (FR); MOKRINI, Manal, 38170 Seyssinet-Pariset (FR)
(74) Representative: Germain Maureau

(57) **Abstract**

This safety device (1) includes a body (2) extending along a longitudinal axis A and configured for receiving a medical container (7) provided with an injection needle (72) and a plunger rod (75), a needle guard (3) axially movable with respect to the body (2) between an initial position and a safety position distally located with respect to the initial position, biasing means for moving the needle guard (3) from the initial position to the safety position, a retainer (5), movable between a blocking position and a release position. The retainer (5) includes a camming surface (53) configured for abutting against the plunger rod (75) such that distal movement of the plunger rod (75) causes movement of the retainer (5) from the blocking position to the release position, and a distal blocking surface (52) configured for axially abutting against a proximal abutment surface (25) of the body (2) when the retainer (5) is in the initial position. One of the distal blocking surface (52) and the proximal abutment surface (25) has a convex shape while the other has a concave shape.

## Description

The present invention relates to an anti-needle stick safety device configured for receiving a medical container and for protecting a user against needle stick injuries, and an injection device including this safety device.

In this application, the distal end of a component or of a device is to be understood as meaning the end furthest from the user's hand and the proximal end is to be understood as meaning the end closest to the user's hand. Likewise, in this application, the "distal direction" is to be understood as meaning the direction away from the user's hand, and the "proximal direction" is to be understood as meaning the direction toward the user's hand.

It is known to use safety devices for a medicine cartridge comprising a body for receiving a cartridge, a guard for covering a used needle, a plunger to dispense medicine from the cartridge and a spring for activation of the guard. The guard and the body are held together by two trigger fingers prior to use of the device.

The trigger fingers include a distal surface which is totally flat and which abuts against a flat seat of the body. These two flat surfaces contact with each other to maintain the guard in its initial position. However, the distal surface of the trigger fingers and the seat of the body do not allow for proper positioning of the trigger fingers with respect to the seat. The proper positioning of the trigger fingers of the state of the art depends on how the body and the guard are assembled to each other.

Besides, the trigger fingers are typically molded on both sides of a mold cavity. This may result in some defects, such as slight offsets on the distal surface of the trigger fingers, misalignment of the trigger fingers with respect to the body, reduction of the contact surface between the trigger fingers and the body, etc. These small defects may be detrimental to the clipping function of the trigger fingers and more generally to the proper operation of the safety device.

There is therefore a need for a safety device that alleviate some or all of the aforementioned drawbacks of the prior art and that permits to reliably hold the guard and the body together prior to use of the safety device. More specifically, there is a need for a safety device ensuring proper positioning of the trigger fingers with respect to the body.

In this context, an aspect of the invention is a safety device configured for protecting a user against needle stick injuries, the safety device including:
a body extending along a longitudinal axis A and configured for receiving a medical container provided with an injection needle and a plunger rod,
a needle guard axially movable with respect to the body between an initial position and a safety position distally located with respect to the initial position
biasing means for moving the needle guard from the initial position to the safety position,
a retainer, movable between a blocking position in which the retainer blocks the needle guard in the initial position against the action of the biasing means, and a release position in which the retainer no longer prevents the biasing means from moving the needle guard to the safety position, the retainer including
a camming surface configured for abutting against the plunger rod such that distal movement of the plunger rod causes movement of the retainer from the blocking position to the release position, and
a distal blocking surface configured for axially abutting against a proximal abutment surface of the body when the retainer is in the initial position, wherein
one of the distal blocking surface and the proximal abutment surface has a convex shape while the other has a concave shape.

The safety device of the invention thus allows for auto-centering of the retainer with respect to the body. This results in a more efficient contact between the retainer and the body, thereby improving the reliability of the safety device in terms of preventing inadvertent activation.

By a surface having concave or convex shape it is meant that the surface is not merely planar and thus does not extend in a single plane. Instead, the surface has a relief, either in the form of a protrusion or rib, or in the form of a recess or groove, and the surface is shaped to converge towards this relief, for instance by means of slanted walls.

The device of the invention may further include some or all of the features listed below.

In an embodiment, the distal blocking surface includes a relief extending at a center of the distal blocking surface.

The distal blocking surface may include a single relief.

The same applies to the proximal abutment surface.

In an embodiment, the distal blocking surface of the retainer includes two inclined surfaces defining a crest or a talweg.

The crest or talweg longitudinally extends in a vertical plane including the central longitudinal axis A. The crest or talweg is the relief of the distal blocking surface.

In an embodiment, the crest or talweg is arranged at the middle of the distal blocking surface of the retainer.

The crest or talweg thus extends in a plane that may divide the retainer in two symmetrical portions.

In an embodiment, each one of the two inclined surfaces is flat.

The above features may also apply to the proximal abutment surface of the body.

In an embodiment, the two inclined surfaces have a same inclination angle with respect to a horizontal plane orthogonal to the longitudinal axis A.

In an embodiment, the inclination angle is greater or equal to 15°, preferably greater or equal to 22° or 23°.

In an embodiment, the retainer includes orthoradial extensions.

In an embodiment, the retainer has a constant width.

The width may be constant from a proximal end to a distal end of the retainer, apart from the orthoradial extensions.

In an embodiment, the retainer has a decresing thickness in the distal direction.

In an embodiment, the distal blocking surface of the needle guard and the proximal abutment surface of the body are complementarily shaped.

Another aspect of the invention is an injection device including a medical container provided with an injection needle and a plunger rod, the medical container being arranged in the body of the aforementioned safety device.

The invention and the advantages arising therefrom will clearly emerge from the detailed description that is given below with reference to the appended drawings as follows :
- Figure 1 is an exploded view of an injection device according to an embodiment of the invention,
- Figure 2 is a cross-section view of a safety device according to an embodiment of the invention,
- Figure 3 is a perspective view of a body of a safety device according to an embodiment of the invention,
- Figure 4 is perspective view of a needle guard of a safety device according to an embodiment of the invention,
- Figure 5 is a partial perspective view of a safety device according to an embodiment of the invention,
- Figure 6A is a partial side view of a needle guard of a safety device according to an embodiment of the invention,
- Figure 6B is a partial side view, in a plane orthogonal to the plane of Figure 6A, of a needle guard of a safety device according to an embodiment of the invention,
- Figure 7 is a schematic view of a trigger finger and a body of a safety device according to an embodiment of the invention,
- Figures 8A to 8E illustrate auto-centering of a trigger finger with respect to a body of a safety device according to an embodiment of the invention,
- Figures 9A to 9F illustrate operation steps of an injection device according to an embodiment of the invention.

The different features of the embodiments can be used in combination with and used with other embodiments as long as the combined parts are not inconsistent with or interfere with the operation of the device and assembly. This invention is not limited in its application to the details of construction and the arrangement of components set forth in the following description or illustrated in the drawings. The embodiments herein are capable of being modified, practiced or carried out in various ways. The phraseology and terminology used herein is for the purpose of description and should not be regarded as limiting. The use of "including," "comprising," or "having" and variations thereof herein is to encompass the items listed thereafter and equivalents thereof as well as additional items. Unless limited otherwise, the terms "connected," "coupled," and "mounted," and variations thereof herein are used broadly and encompass direct and indirect connections, couplings, and mountings. In addition, the terms "connected" and "coupled" and variations thereof are not limited to physical or mechanical connections or couplings. In contrast, when an element is referred to as being "directly connected" or "directly coupled" to another element, there are no intervening elements present. Unless otherwise defined, all terms (including technical and scientific terms) used herein have the same meaning as commonly understood by one of ordinary skill in the art to which this disclosure belongs. It will be further understood that terms used herein should be interpreted as having a meaning consistent with their meaning in the context of this specification and the relevant art and will not be interpreted in an idealized or overly formal sense unless expressly so defined herein. Further, terms such as distal, proximal, up, down, bottom, and top are relative, and are to aid illustration, but are not limiting. Relative terms such as "below" or "above" or "upper" or "lower" or "horizontal" or "vertical" may be used herein to describe a relationship of one element to another element as illustrated in the Figures. It will be understood that these terms and those discussed above are intended to encompass different orientations of the device in addition to the orientation depicted in the Figures. The embodiments are not intended to be mutually exclusive so that the features of one embodiment can be combined with other embodiments as long as they do not contradict each other. Terms of degree, such as "substantially", "about" and "approximately" are understood by those skilled in the art to refer to reasonable ranges around and including the given value and ranges outside the given value, for example, general tolerances associated with manufacturing, assembly, and use of the embodiments. The term "substantially" when referring to a structure or characteristic includes the characteristic that is mostly or entirely present in the structure. As used herein, the singular forms "a," "an," and "the" are intended to include the plural forms as well, unless the context clearly indicates otherwise. As used herein, the term "and/or" includes any and all combinations of one or more of the associated listed items. It will be understood that, although the terms first, second, etc., may be used herein to describe various elements, these elements should not be limited by these terms. These terms are only used to distinguish one element from another. For example, a first element could be termed a second element, and, similarly, a second element could be termed a first element without departing from the scope of the present disclosure. For simplification, the parts or elements of one embodiment which are found identically or similarly in the other embodiment will be identified using the same numerical references and will not be described again.

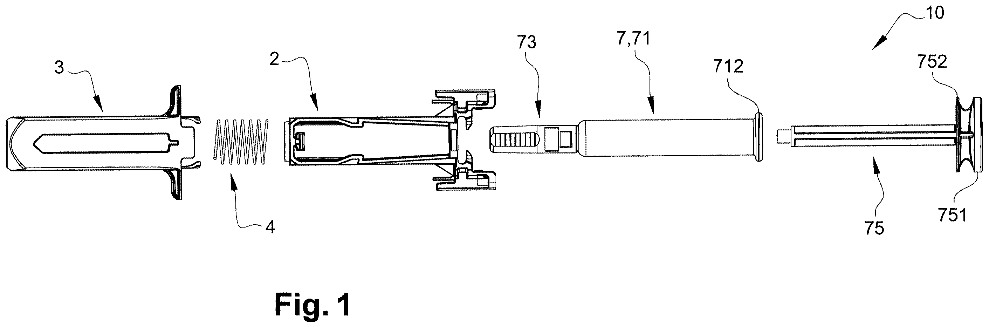

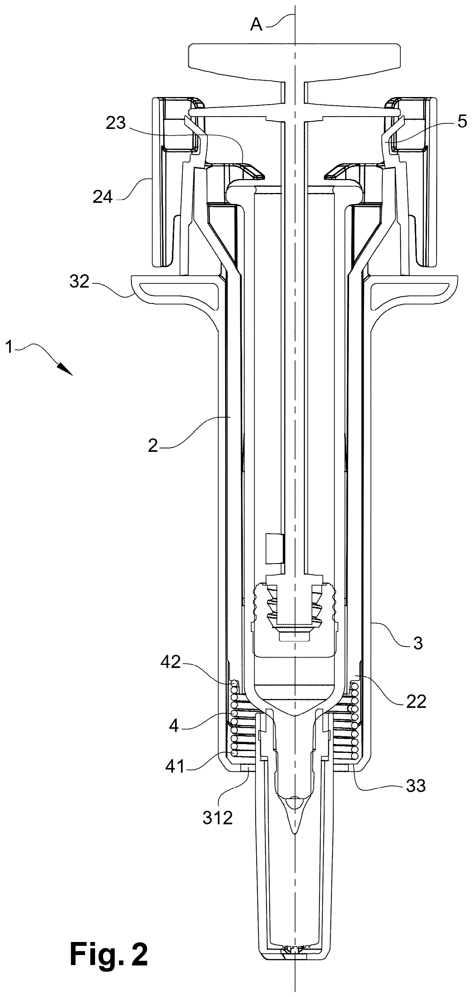

With reference to Figures 1 and 2 are shown an injection device 10 and a safety device 1 according to an embodiment of the invention. The safety device 1 is configured for receiving a medical container 7 and for protecting a user against needle stick injuries after injection.

The safety device 1 includes a body 2 for accommodating a medical container 7 and a needle guard 3 for protecting against needle stick injuries. The body 2 may be arranged within the housing. The needle guard 3 is axially movable with respect to the housing between an initial (retracted) position, in which the needle guard 3 allows for performing an injection operation, and a safety (extended) position, distally located with respect to the initial position, in which the needle guard 3 is configured to distally protrude from the body 2 to prevent needle stick injuries.

The safety device 1 further includes biasing means, such as a safety spring 4, for moving the needle guard 3 in the distal direction to the safety position with respect to the housing, and one or more retainers 5 for retaining the needle guard 3 in the initial position with respect to the housing. The safety spring 4 may be initially compressed between the body 2 and the needle guard 3. The retainers 5 and the needle guard 3 may be made of a single piece.

The safety device 1 may further include a locker for locking the needle guard 3 in the safety position. The locker and the needle guard 3 may be made of a single piece.

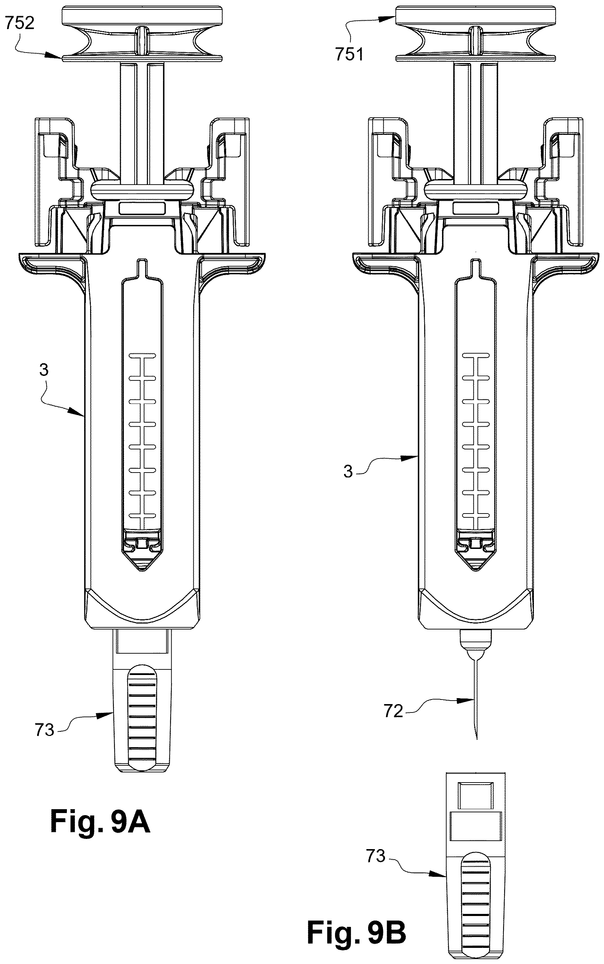

With reference to Figure 1, the medical container 7, may be a prefillable or prefilled syringe, which may include a tubular barrel 71 defining a reservoir configured for containing a medical product. The tubular barrel 71 may include an elongated distal tip 711 defining an axial passageway for passage of the medical product from the reservoir and an injection needle 72, Figure 9B, attached to this distal tip. Prior to use of the injection device 10, the injection needle 72 is sealed and protected by a removable needle shield 73. A stopper 74 is arranged within the barrel 71 for pushing fluid outside the barrel 71 via the distal tip and the injection needle 72. At its proximal end, the barrel 71 may include a radial flange 712 which may serve to hold the medical container 7 at a fixed axial position within the body 2. The radial flange 712 delimits a distal side configured for abutting against the housing. The barrel 71 may include a glass or a plastic material. The medical container 7 further includes a plunger rod 75 extending through the opened proximal end of the barrel 71 to push the stopper 74 in the distal direction to perform injection. The plunger rod 75 includes a plunger head 751 allowing the user to use his/her thumb to distally press against the plunger rod 75, and a trigger member 752, which may be a distal side of the plunger head 751 or which may be proximal to the plunger head 751, for engaging the retainer, i.e. the trigger fingers, in order to release the needle guard 3 such that the needle guard 3 can move towards the safety position.

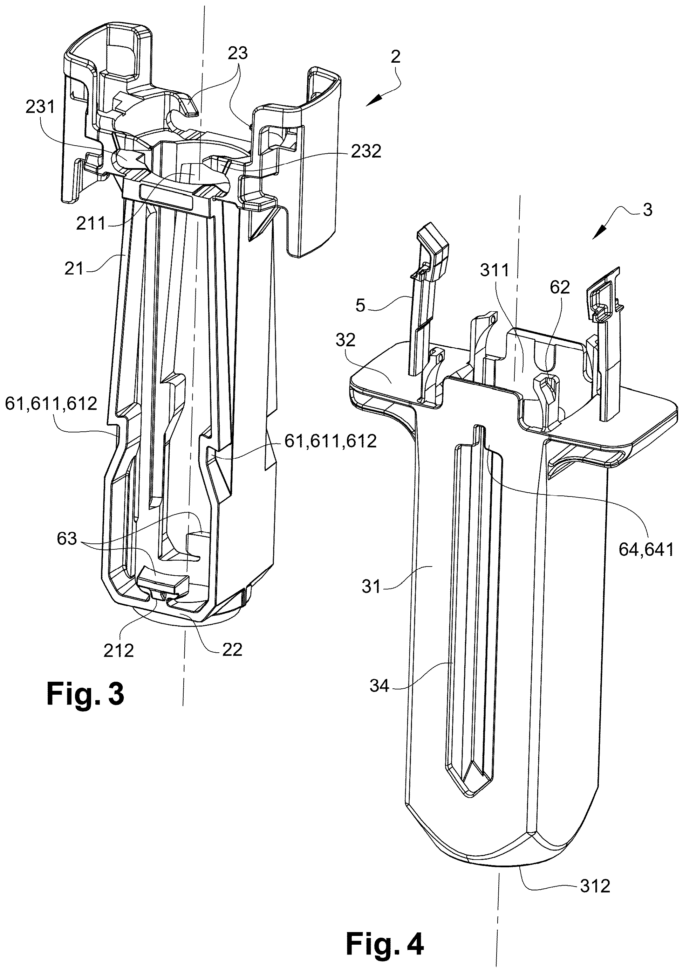

With reference to Figures 2 and 3, the body 2 includes a tubular lateral wall 21 longitudinally extending along a central longitudinal axis A and defining an inner cavity for receiving the medical container 7, a proximal end delimiting a proximal opening 211 leading into the inner cavity for allowing insertion of the medical container 7 therein, and a distal end delimiting a distal opening 212 for allowing extension of the injection neede and the needle shield 73 outside the body 2.

The body 2 further includes a distal seat 22 which may be the distal end of the body 2 for providing support to the safety spring 4, and snap-fit members 23 for securing the medical container 7 inside the body 2.

The snap-fit members 23 may be resiliently deformable arms configured for resiliently deflecting in order to allow insertion of the flange of the medical container 7 therebteween and to move back towards their initial position to capture the flange of the medical container 7 between a proximal stop 231 of the body 2 and a distal stop 232 defined at a distal side of the resiliently deformable arms. As a result, the medical container 7 is axially secured with respect to the body 2.

The body 2 may include two diametrically opposite protections 24 that may extend around the retainers 5 in order to protect them. Therefore, risks of inadvertent movement of the retainers 5 prior to use of the device are limited. The protections 24 may proximally extend beyond the trigger fingers, and may distally extend beyond the proximal stop 231 of the body 2. The opposite protections 24 may help align and center the plunger head 751 into the device activation area of the safety device 1.

The body 2 further includes a proximal abutment surface 25 for axially abutting against the retainers 5 in order to initially block the needle guard 3 in the initial position.

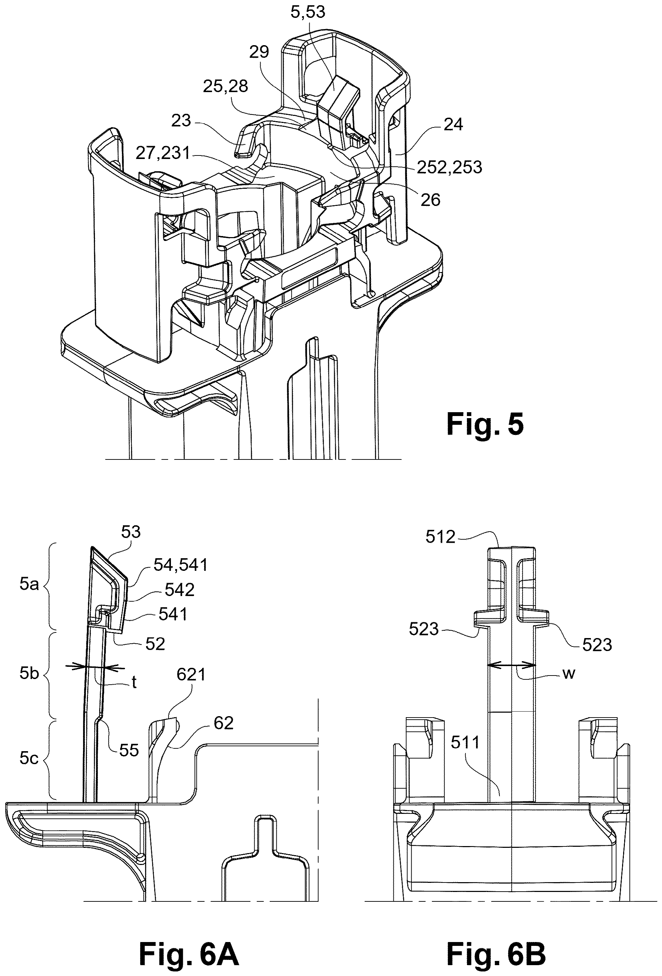

With reference to Figure 5, the proximal abutment surface 25 may be arranged at the proximal end of the body 2, on a partial enclosing wall 26 configured to guide and center the flange of the medical container 7 with respect to the body 2. The enclosing wall 26 may connect two adjacent resiliently deformable arms. The enclosing wall 26 proximally protrudes from a distal shoulder 27 which delimits the distal stop 232 abutting against the flange of the medical container 7.

The proximal abutment surface 25 may be a bottom of a cutout 28 further including two orthoradial stops 29.

The proximal abutment surface 25 is not orthogonal to the longitudinal axis A, and is not totally included in a single plane. Instead, the proximal abutment surface 25 has a convex shape. This convex shape helps center the retainer, as will be explained in further details below. Thus, the proximal abutment surface 25 distally tapers.

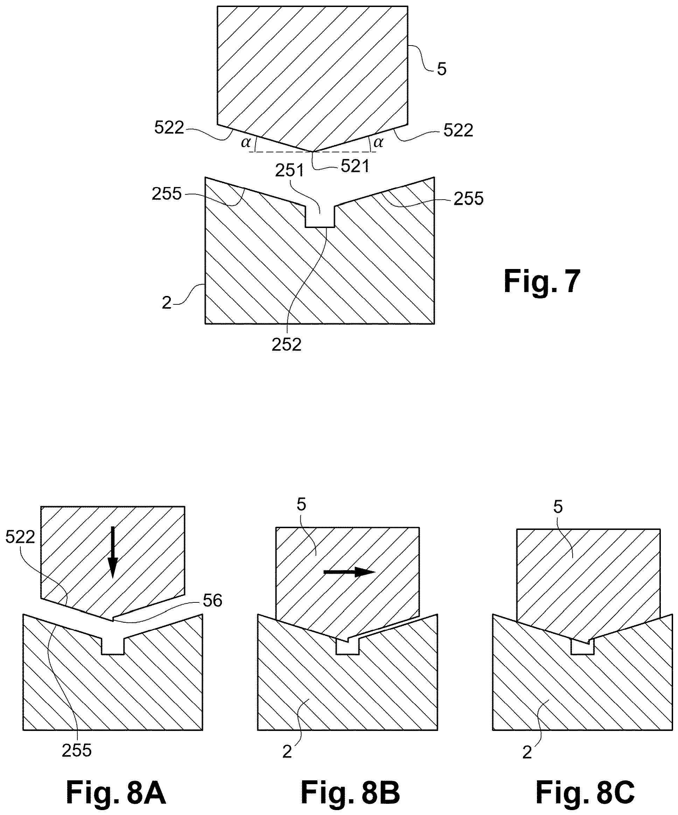

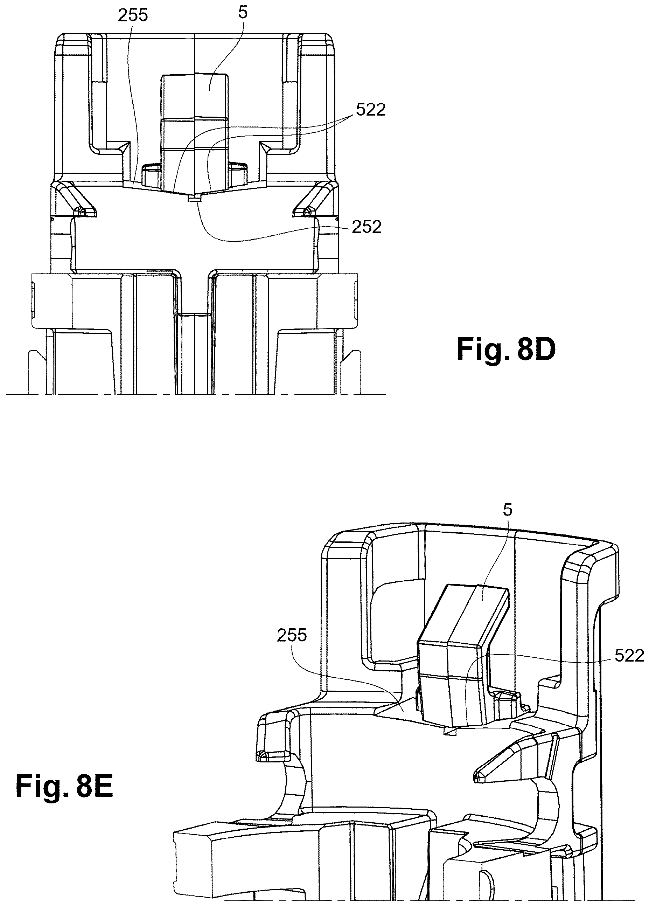

With reference to Figures 7 and 8A-8C, the proximal abutment surface 25 includes a relief 251, such as bottom or a talweg, which may be arranged at the middle of the proximal abutment surface 25. The bottom or talweg 251 of the proximal abutment surface 25 is preferably located in the longitudinal plane including the finger flanges 32 of the needle guard 3 and dividing the safety device 1 in two symmetrical parts. The proximal abutment surface 25 includes a single relief 251, and the surfaces next to it converge towards this relief 251.

The proximal abutment surface 25 and the retainer 5 may have complementary shapes.

The bottom may include a groove 252 for receiving any burr that the retainer 5 may have. The groove 252 may be a radial groove, and may have an opened centripetal end 253, Figure 5, and/or an opened centrifugal end 254.

In the embodiment illustrated in Figures 7 and 8A-8C, the proximal abutment surface 25 is formed by two inclined surfaces 255, inclined toward each other and toward the bottom of the proximal abutment surface 25 which is substantially at the middle of the proximal abutment surface 25. The two inclined surfaces 255 therefore intersect each other at the bottom 251 of the proximal abutment surface 25.

As visible in Figure 6A, it should be noted that the proximal abutment surface 25, and its two inclined surfaces 255, may further also be inclined away from the central longitudinal axis A to prevent inadvertant outward radial movement of the retainer 5 and thus to better keep the retainer 5 in the blocking position. Therefore, the inclined surfaces 255 have a double inclination : a first orthoradial inclination towards the other inclined surface and towards the bottom, and a second radially inward inclination towards the retainer 5.

The two inclined surfaces 255 may have the same angular inclinations ; they may be symmetrical with respect to a median longitudinal plane dividing the body 2 in two similar halves.

With reference to Figure 4, the needle guard 3 includes a tubular lateral wall 51 defining an inner cavity for receiving the body 2, a finger flange 32 for providing support to the user's fingers, and one or more retainers 5 in the form of two diametrically opposite trigger fingers.

The tubular wall of the needle guard 3 has an opened proximal end 311 for allowing insertion of the body 2 therethrough, and an opened distal end 312 for allowing passage of the needle shield 73 prior to use of the injection device 10 and allowing the injection needle 72 to distally extend over the needle guard 3 during injection.

A proximal seat 33 may be arranged at the distal end of the needle guard 3 to provide support for a distal end of the safety spring 4.

As visible in figure 2, the safety spring 4 has a distal end 41 axially abutting against the proximal seat 33 of the needle guard 3, a proximal end 42 axially abutting against the distal end of the body 2. Therefore the safety spring 4 is located outside the body 2, and is rather lodged inside the needle guard 3, in a distal portion of the needle guard 3, between the distal end of the body 2 and the distal end of the needle guard 3. The safety spring 4 may be a coil spring definning an inner passage allowing for extension of the medical container 7 therethrough.

With reference to Figures 6A-6B, the retainers 5 may be in the form of two diametrically opposite trigger fingers. The trigger fingers 5 may extend parallel to the longitudinal axis A or slightly inwardly inclined with respect to the longitudinal axis A, and may proximally protrude from the finger flange 32 of the needle guard 3.

The trigger fingers 5 have a distal end 511, which may be fixed to the finger flange 32 of the needle guard 3, and a proximal end 512, which may be free.

The proximal end 511 of the trigger fingers 5 extends proximally beyond the body 2 so that the trigger member 752 of the plunger rod 75 can abut against the trigger fingers 5 during injection.

The trigger fingers 5 include a distal blocking surface 52 configured for axially abutting against the body 2 in order to block the needle guard 3 in its initial position, and a camming surface 53 configured for abutting against the plunger rod 75 in order to cause disengagement of the distal blocking surface 52 of the trigger fingers 5 from the body 2 at the end of injection.

The trigger fingers 5 may further include a sliding side surface 54 which may be radially convex and which may be composed by two slanted walls 541 delimiting a radially inward protrusion 542.

The camming surface 53 is inclined with respect to the longitudinal axis A and tapers in a distal direction. The camming surface 53 may extend from the proximal end of the trigger fingers 5, and possibly (but not necessarily) to the distal blocking surface 52.

The distal blocking surface 52 is not flat, is not formed by a single planar surface, and does not extend orthogonal to the longitudinal axis A. Instead, the distal blocking surface 52 has a convex shape configured to fit the concave shape of the proximal abutment surface 25 of the body 2. This allows for auto-centering of the trigger fingers 5 when the trigger fingers 5 abut against the body 2 and are submitted to the pressure of the safety spring 4. Instead of a distal blocking surface 52 having a convex shape, the distal blocking surface 52 could have a concave shape and the corresponding proximal abutment surface 25 of the body 2 could have a convex shape in order to engage the concave shape of the trigger finger 5.

In an embodiment, the distal blocking surface 52 has a relief 521, such as an apex or a crest, for engaging the bottom or talweg 251 of the proximal abutment surface 25 of the body 2. The distal blocking surface 52 distally tapers. The apex or crest 521 may extend at the middle of the distal blocking surface 52. The bottom or talweg 251 of the proximal abutment surface 25 is preferably located in the longitudinal plane including the finger flanges 32 of the needle guard 3 and dividing the safety device 1 in two symmetrical parts. The distal blocking surface 52 includes a single relief 521, and the surfaces next to it converge towards this relief 521.

The distal blocking surface 52 may include two inclined surfaces 522 intersecting at the crest 521. The two inclined surfaces 522 may be planar. They are inclined in an orthoradial direction, i.e. with respect to a horizontal plane orthogonal to the longitudinal axis A.

The two inclined surface 522 may also be inclined in the radial direction, such that the distal blocking surface 52 forms an acute angle α, Figure 7, with the trigger finger 5. This limits risks of inadvertent activation of the trigger finger 5. Therefore, the inclined surfaces 522 have a double inclination : a first orthoradial inclination towards the crest 521, and a second radially inward inclination towards the central longitudinal axis A.

The two inclined surfaces 522 may have the same angular inclinations ; they may be symmetrical with respect to a longitudinal median plane dividing the trigger finger 5 in two similar halves.

As illustrated in Figure 7, the angle α of the planar surfaces 522 may be greater or equal to 15°, preferably greater or equal to 20°, even preferably greater or equal to 22° or 23°.

Instead of being formed by two inclined surfaces 522, the distal blocking surface 52 of the trigger finger 5 and the proximal abutment surface 25 of the body 2 could be, for instance, conical.

The distal blocking surface 52 may include two orthoradial extensions 523, that orthoradially protrude from each side of the trigger fingers 5 to increase the contact surface with the body 2. Apart from the portion where the extensions 523 are located, the trigger finger 5 may otherwise have a constant width w.

The trigger fingers 5 may have a proximal portion 5a including the camming surface 53, the sliding surface 54, and the distal blocking surface 52, a distal portion 5c fixedly connected to the needle guard 3, and an intermediate portion 5b extending between the proximal portion 5a and the distal portion 5c. The thickness t of the proximal portion 5a may be greater than the thickness of the intermediate portion 5b, which itself may be greater than the thickness of the distal portion 5c. The trigger fingers 5 may include a distal shoulder 55 arranged on an inner side of the trigger fingers 5 and separating the intermediate portion 5b from the distal portion 5c.

The trigger fingers 5 may be resiliently deformable.

The trigger fingers 5 are radially movable between an initial blocking position, in which the distal blocking surface 52 of the trigger fingers 5 axially abuts against the proximal abutment surface 25 of the body 2 to maintain the needle guard 3 in its initial position against the force of the safety spring 4, and a release position, radially outwardly located with respect to the blocking position, in which the distal blocking surface 52 is shifted away from and no longer abuts against the proximal abutment surface 25 of the body 2 such that the needle guard 3 can move with respect to the body 2 towards its safety position under the force of the safety spring 4. Deformation of the trigger fingers 5 from the blocking position to the release position is caused by the plunger rod 75 abutting against the camming surface 53 of the trigger fingers 5 when the plunger rod 75 is moved in the distal direction during injection.

In an embodiment, the trigger fingers 5 may be made of polycarbonate (PC), while the body 2 may be made of polycarbonate (PC) . The plunger rod 75 may be made of polycarbonate (PC), polypropylene (PP), acrylonitrile butadiene styrene (ABS), or polyoxymethylene (POM) or any combination thereof.

Figures 8A-8C schematically illustrate auto-centering of the trigger fingers. To illustrate a worst case scenario, the trigger finger 5 here has a burr 56 on its distal blocking surface 52. First, during assembly of the body 2 and the needle guard 3, the distal blocking surface 52 of the trigger finger 5 is put into contact with the proximal abutment surface 25 of the body 2, Figure 8A. Due to the force exerted by the safety spring 4 on the needle guard 3, and to the cooperation of the slanted walls of the trigger fingers 5 and the slanted walls of the body 2, the trigger finger 5 automatically aligns, Figures 8B-8C. The contact is efficient despite the burr, because the burr 56 is received in the groove 252 of the proximal abutment surface 25 of the body 2, thereby allowing a two-plane contact between the trigger finger 5 and the body 2. That is, a first of the two slanted walls 522 of the trigger finger 5 abuts againts a first of the two slanted walls 255 of the body 2, and a second of the two slanted walls 522 of the trigger finger 5 simultaneously abuts against a second of the two slanted walls 255 of the body 2. Since the slanted walls 522 of the trigger finger 5 (and so are the slanted walls 255 of the body 2) are angled with respect to each other, the trigger finger 5 is well centered.

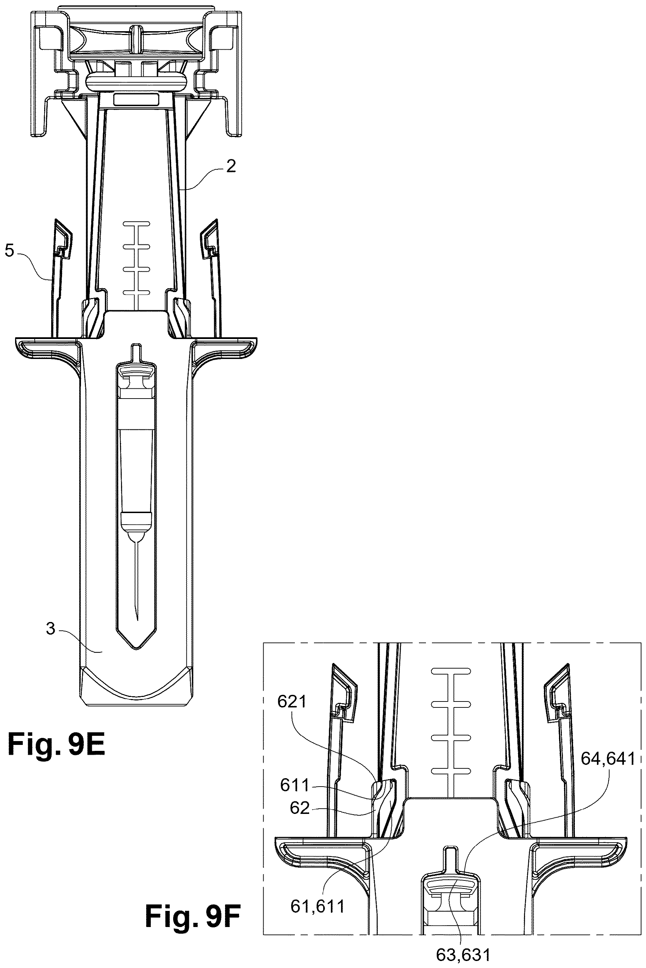

With reference to Figure 9F, the locker includes a first locking member 61, which may be arranged on the body 2, and a second locking member 62, which may be arranged on the needle guard 3, configured to engage with each other in order to axially block the needle guard 3 in its safety position.

The first locking member 61 may include a distal blocking surface 611 defined by a radial recess 612 arranged on an outer surface of the lateral wall of the body 2, at a distal end thereof. The distal blocking surface 52 blocks any axial movement of the needle guard 3 in the proximal direction.

The second locking member 62 may be a resilient leg, proximally protruding from a proximal side of the finger flanges 32, and shaped to engage the radial recess 612 when the needle guard 3 reaches the safety position, such that a proximal blocking surface 621 of the second blocking member abuts against the distal blocking surface 52 of the first locking member 61.

The locker may further include a third locking member 63, which may be arranged on the body 2, and a fourth locking member 64, which may be arranged on the needle guard 3, configured to engage with each other in order to axially block the needle guard 3 in its safety position.

The third locking member 63 may include a proximal blocking surface 631 defined by a tab arranged at a distal end of the body 2. The fourth locking member 64 may include a distal blocking surface 641 arranged at a proximal end of the window 34 extending through the lateral wall of the needle guard 3, and configured to axially abut against the third locking member 63 when the needle guard 3 reaches the safety position, such that the needle guard 3 cannot move further in the distal direction.

In other embodiments not shown, the locker may include only the first locking member 61 and the second locking member 62, or only the third locking member 63 and the fourth locking member 64, each of these locking members including both a proximal blocking surface and a distal blocking surface engaging a corresponding distal blocking surface and a corresponding proximal blocking surface of the other locking member in order to axially lock the needle guard 3 in the safety position.

The invention also relates to an injection device 10 including a medical container 7 and the above-described safety device 1 accommodating this medical container 7.

The safety device 1 of the invention thus allows for auto-centering, and consequently for proper positioning of the trigger fingers 5 with respect to the body 2. This results in a more reliable contact between the trigger fingers 5 and the body 2, thus efficiently preventing inadvertent activation of the safety device 1 or any dysfunction during use of the injection device 10.

With reference to Figures 9A-9F, operation of the injection device 10 of the invention is described below.

The user may first assemble the injection device 10 to the safety device 1, by introducing the injection device 10 through the opened proximal end of the body 2, until the flange of the medical container 7 is captured by the resilient arms of the body 2, Figure 9A.

In order to perform an injection operation, the user first removes the needle shield 73, Figure 9B, thereby exposing the injection needle 72. The user then inserts the injection needle 72 into an injection site and presses against the plunger head 751 such that the plunger rod 75 pushes the stopper 74 in the distal direction. The medical product contained within the barrel 71 is therefore expelled towards the injection site via the injection needle 72.

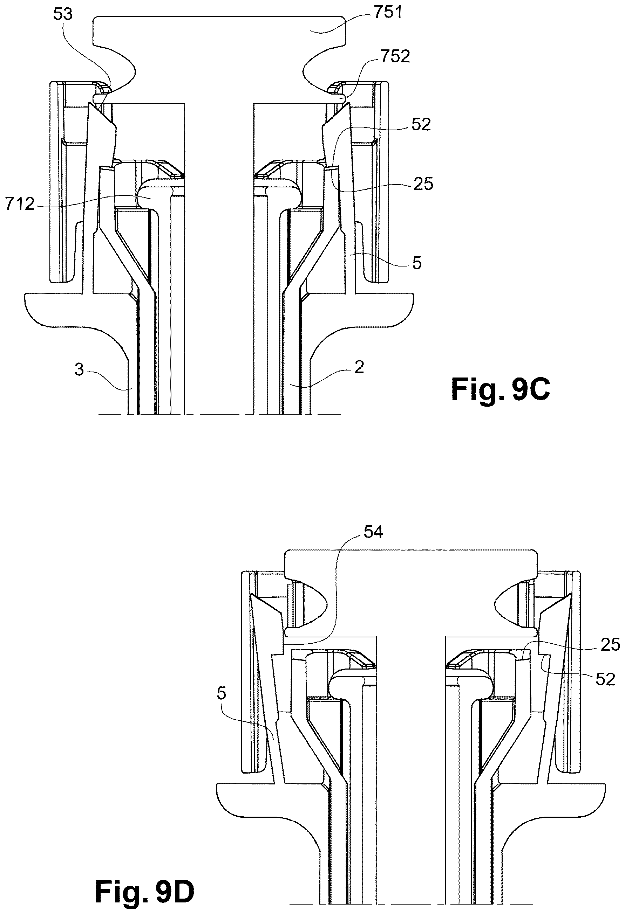

At the end of injection, the trigger member 752 of the plunger rod 75 abuts against the trigger fingers 5 of the needle guard 3, Figure 9C. Further movement of the plunger rod 75 in the distal direction causes the trigger fingers 5 to move radially outwardly, from their blocking position to their release position, Figure 9D. As a result, the needle guard 3 can now move in the distal direction under the force of the safety spring 4, till the safety position in which the needle guard 3 surrounds and forbids access to the injection needle 72, Figure 9E. Due to the locker, the needle guard 3 cannot move back in the proximal direction, Figure 9F. The safety device 1 accordingly protects the user against needle stick injuries.

It is readily understandable from the above description that the safety device 1 of the invention allows for auto-centering, and consequently for proper positioning of the trigger fingers 5 with respect to the body 2. This results in a more reliable contact between the trigger fingers 5 and the body 2, thus efficiently preventing inadvertent activation of the safety device 1 or any dysfunction during use of the injection device 10. It is to be understood that the present invention is not limited to the embodiments described above and illustrated in the drawings; rather, the skilled person will recognize that many changes and modifications may be made within the scope of the appended claims.

## Claims

1. Safety device (1) configured for protecting a user against needle stick injuries, the safety device (1) including:
a body (2) extending along a longitudinal axis A and configured for receiving a medical container (7) provided with an injection needle (72) and a plunger rod (75),
a needle guard (3) axially movable with respect to the body (2) between an initial position and a safety position distally located with respect to the initial position
biasing means for moving the needle guard (3) from the initial position to the safety position,
a retainer (5), movable between a blocking position in which the retainer (5) blocks the needle guard (3) in the initial position against the action of the biasing means, and a release position in which the retainer (5) no longer prevents the biasing means from moving the needle guard (3) to the safety position, the retainer (5) including
a camming surface (53) configured for abutting against the plunger rod (75) such that distal movement of the plunger rod (75) causes movement of the retainer (5) from the blocking position to the release position, and
a distal blocking surface (52) configured for axially abutting against a proximal abutment surface (25) of the body (2) when the retainer (5) is in the initial position, wherein
one of the distal blocking surface (52) and the proximal abutment surface (25) has a convex shape while the other has a concave shape.

2. Safety device (1) according to the preceding claim, wherein the distal blocking surface (52) includes a relief (521) extending at a center of the distal blocking surface (52).

3. Safety device (1) according to any of the preceding claims, wherein the distal blocking surface (52) of the retainer (5) includes two inclined surfaces (522) defining a crest or a talweg (521).

4. Safety device (1) according to the preceding claim, wherein the crest or talweg (521) is arranged at the middle of the distal blocking surface (52) of the retainer (5).

5. Safety device (1) according to any of the preceding claims 3-4, wherein each one of the two inclined surfaces (522) is flat.

6. Safety device (1) according to any of the preceding claims 3-5, wherein the two inclined surfaces (522) have a same inclination angle with respect to a horizontal plane orthogonal to the longitudinal axis A.

7. Safety device (1) according to the preceding claim, wherein the inclination angle is greater or equal to 15°, preferably greater or equal to 22° or 23°.

8. Safety device (1) according to any of the preceding claims, wherein the retainer (5) includes orthoradial extensions (523).

9. Safety device (1) according to any of the preceding claims, wherein the retainer (5) has a constant width (w).

10. Safety device (1) according to any of the preceding claims, wherein the retainer (5) has a decresing thickness (t) in the distal direction.

11. Safety device (1) according to any of the preceding claims, wherein the distal blocking surface (52) of the needle guard (3) and the proximal abutment surface (25) of the body (2) are complementarily shaped.

12. Injection device (10) including a medical container (7) provided with an injection needle (72) and a plunger rod (75), the medical container (7) being arranged in the body (2) of a safety device (1) according to any of the preceding claims.
